# FASCICULE DE BREVET EUROPEEN

(11) **EP 0 998 903 B1**
(45) Date de publication et mention de la délivrance du brevet: **08.09.2004**
(21) Numéro de dépôt: 99402573.2
(22) Date de dépôt: 19.10.1999
(51) Int. Cl.: A61K 7/00

(54) **Patch, ensemble constitué d'un patch et d'un récipient, et procédé**
Pflaster, Anordnung bestehend aus einem Pflaster und einem Behälter, und Verfahren
Patch, assembly consisting of a patch and a container, and process

(30) Priorité: 20.10.1998 FR 9813129
(43) Date de publication de la demande: 10.05.2000
(73) Titulaire: L'OREAL, 75008 Paris (FR)
(72) Inventeur: Gueret, Jean-Louis, 75016 Paris (FR)
(74) Mandataire: Leszczynski, André

(56) Documents cités:
- EP-A- 0 674 913
- EP-A- 0 764 441

## Description

La présente invention concerne un patch destiné à être appliqué sur la peau pour effectuer un traitement déterminé, par exemple un traitement cosmétique avec libération de substances actives destinées à exercer sur l'épiderme une action donnée.

On connaît de nombreux patchs de ce type, lesquels présentent d'une manière générale une structure multicouche comprenant une matrice adhésive à base d'un ou plusieurs polymères, destinée à venir directement au contact de la peau par une face et accolée par l'autre face à un support.

L'invention concerne plus particulièrement les patchs dans lesquels la matrice présente des propriétés adhésives à l'état sec.

Dans de tels patchs, la matrice contient avantageusement une ou plusieurs substances actives destinées à être libérées sur l'épiderme pour exercer un effet donné, par exemple une action hydratante ou régénérante.

La libération de ces substances actives peut être obtenue en mouillant la peau préalablement à l'application du patch.

La demande de brevet EP 0 674 913 décrit un patch imprégné d'un gel contenant un ou plusieurs médicaments aussi qu'un humidifiant.

En variante, l'eau servant à faire passer en solution les substances actives, contenues notamment à la surface de la matrice peut provenir de la région de peau située sous le patch, auquel cas il est préférable d'utiliser un support occlusif de manière à favoriser la transpiration locale. Un patch occlusif est décrit dans la demande de brevet EP 0 764 441.

Les patchs connus n'offrent pas entière satisfaction.

En particulier, la quantité d'eau déposée sur la peau avant l'application du patch peut s'avérer insuffisante pour assurer la solubilisation recherchée des substances actives contenues dans la matrice.

En outre, l'utilisation d'un support occlusif peut ne pas être très confortable, procurant un effet chauffant plutôt que rafraîchissant.

Il existe par conséquent un besoin pour améliorer encore les conditions d'application d'une ou plusieurs substances actives sur la peau au moyen d'un patch.

La présente invention a pour objet un nouveau patch, du type comportant une matrice présentant des propriétés adhésives à l'état sec et contenant au moins une substance active, cette matrice étant accolée par une face à un support et étant à appliquer par l'autre face sur la peau, ce patch étant caractérisé par le fait que ledit support est choisi de manière à pouvoir contenir un liquide prédéterminé apte à solubiliser au moins partiellement la ou les substances actives contenues dans la matrice et par le fait que la matrice est perméable à ce liquide.

Ainsi, grâce à l'invention, le liquide qui peut être de l'eau ou tout autre solvant peut diffuser vers la peau à travers la matrice, depuis le support qui constitue alors en quelque sorte un réservoir, ce qui présente de multiples avantages.

Tout d'abord, au cours de sa diffusion à travers la matrice le liquide provoque le passage en solution de la ou des substances actives précitées, lesquelles sont ainsi libérées de la matrice.

La matrice peut agir à la manière d'une "mèche" en tendant à absorber, par capillarité, le liquide imprégnant le support, ainsi qu'à la manière d'un "filtre" en retenant des particules en son sein tout en permettant la traversée des substances actives solubilisées.

De plus, en particulier lorsque le liquide est de l'eau et que le patch est appliqué sur la peau mouillée, le support imprégne d'eau permet de retarder le séchage à l'interface avec la peau et se comporte ainsi en quelque sorte comme un support occlusif, sans toutefois présenter les inconvénients spécifiques à l'utilisation d'un tel support, notamment en ce qui concerne le confort d'utilisation.

Il peut en outre s'établir une circulation fluidique entre l'interface avec la peau et le support imprégné de liquide, favorable à la respiration de la peau et au renouvellement des substances actives venant au contact avec la peau et/ou à l'élimination vers le support d'impuretés ou de toxines libérées à l'interface avec la peau.

Enfin, l'inertie thermique du support imbibé ainsi que l'évaporation du liquide à sa surface procure à l'utilisateur une sensation de fraîcheur qui accroît encore le confort d'utilisation.

Dans une mise en oeuvre préférée de l'invention, la matrice comporte un ou plusieurs polymères qui peuvent être des élastomères.

Le liquide servant à imprégner le support peut être de l'eau ou une solution aqueuse ou alcoolique, auquel cas la matrice comporte de préférence au moins un polymère hydrophobe.

Le support est avantageusement constitué d'une matière poreuse, par exemple un non-tissé.

La ou les substances actives contenues dans la matrice peuvent être des composés cosmétiquement actifs, de préférence hydrosolubles, et peuvent être dispersés dans la matrice sous une forme particulaire.

Ce ou ces composés cosmétiquement actifs sont choisis par exemple parmi les agents émollients, hydratants, cicatrisants, régénérants, anti-rides, tenseurs, insensibilisants, protecteurs solaires, apaisants, auto-bronzants, éclaircissants, anti-tâches, réducteurs de graisse ou des mèlanges de ceux-ci.

Dans une mise en oeuvre préférée de l'invention, la matrice renferme en outre une dispersion d'une charge de particules d'au moins un composé absorbeur de liquide, par exemple un composé hydrophile absorbeur d'eau lorsque le liquide utilisé est de l'eau ou une solution aqueuse ou alcoolique.

Ce composé absorbeur d'eau est alors choisi par exemple dans la liste suivante : polyacrylates réticulés superabsorbants à fort taux de gonflement dans l'eau, alcool polyvinylique, polymères carboxyvinyliques, dérivés semi-synthétiques de la cellulose, amidons, biogommes, biosaccharides, scléroglucanes, caséine, phytocolloïdes tels que les alginates, gélatine, fibres de coton, gélanes, xanthanes, laponite, primogel, silices ou des ménages de ceux-ci. La charge en composé(s) absorbeur(s) de liquide est par exemple dans une proportion comprise entre 0,2% et 50% en poids, de préférence 1 à 20%, de préférence encore 1 à 10%, par rapport au poids total de la matrice.

De préférence, la teneur de la matrice en composé(s) absorbeur(s) de liquide est choisie de telle sorte que la quantité de liquide capable d'être absorbée par ce ou ces composé(s) soit supérieure ou égale à la quantité de liquide nécessaire à la solubilisation de la ou des substances actives contenues dans la matrice.

La matrice peut comporter des ajours, par exemple des microperforations destinées à rendre la matrice perméable au liquide imbibant le support et/ou à favoriser les échanges gazeux et/ou liquides entre le support et l'interface avec la peau.

La matrice peut être déposée sur le support selon un motif en grille ou analogue.

La matrice peut renfermer au moins une substance active capable de réagir chimiquement avec le liquide utilisé.

L'invention a encore pour objet un ensemble constitué d'un patch tel que défini plus haut et d'un récipient contenant un liquide destiné à imprégner le support auquel est accolée la matrice.

Ce liquide peut comporter au moins une substance active destinée à exercer un effet prédéterminé sur la peau, cosmétique ou non, par exemple un anesthésique.

L'invention a encore pour objet un procédé comportant les étapes consistant à :
- fournir un patch tel que défini plus haut,
- imprégner avant ou après l'application sur la peau le support avec un liquide déterminé.

Dans une mise en oeuvre particulière de ce procédé, préalablement à l'application du patch sur la peau, on mouille cette dernière.

La quantité d'eau imbibant le support par unité de surface du support est de préférence supérieure à la quantité d'eau déposée sur la peau, par unité de surface.

On laisse par exemple le patch sur la peau pendant une durée comprise entre 5 min et 60 min.

D'autres caractéristiques et avantages de la présente invention apparaîtront à la lecture de la description détaillée qui va suivre, d'exemples non limitatifs de mise en oeuvre de l'invention, et à l'examen du dessin annexé sur lequel :
- la figure 1 est une vue schématique en coupe d'un patch conforme à un premier exemple de réalisation de l'invention,
- les figures 2 et 3 illustrent l'utilisation du patch de la figure 1,
- la figure 4 est une vue schématique illustrant un mode de propagation du liquide imbibant le support à travers la matrice.
- la figure 5 est une vue schématique en perspective d'un patch conforme à une variante de réalisation de l'invention.

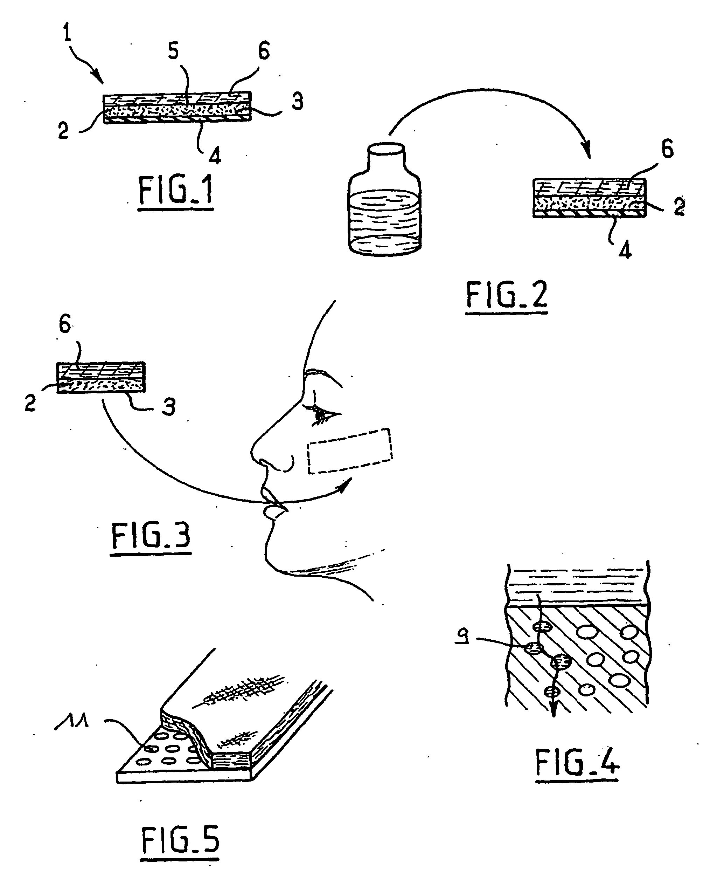

Le patch 1 représenté sur la figure 1 comporte une matrice à base de polymère hydrophobe 2, revêtue sur une face 3 d'une pellicule de protection amovible 4 et accolée par l'autre face 5 à un support poreux 6, constitué dans l'exemple de réalisation décrit par un non-tissé à base de fibres de cellulose.

La matrice 2 est ici réalisée dans un adhésif polyacrylique et présente des propriétés adhésives à l'état sec.

Cette matrice 2 est avantageusement colorée, de manière à permettre à l'utilisateur de visualiser les impuretés susceptibles d'adhérer à la surface du patch, après son enlèvement.

Comme autres matériaux adhésifs hydrophobes utilisables d'une manière générale dans le cadre de la présente invention, on peut citer les adhésifs siliconnés ou polyvinyliques, les polyuréthanes, les élastomères de latex et les élastomères d'EPDM, cette liste étant donnée à titre non limitatif.

La matrice 2 contient dans l'exemple décrit une charge de particules 9 d'un composé hydrophile absorbeur d'eau et renferme en outre des composés cosmétiquement actifs solubles dans le liquide imprégnant le support.

L'épaisseur de la matrice est aisément choisie en fonction de la nature du traitement auquel est destiné le patch, de la nature de la charge et de celle du ou des composés cosmétiquement actifs qu'elle renferme.

L'épaisseur de la matrice 2 est comprise d'une manière générale entre 2 µm et 1,5 mm, étant de préférence comprise 5 µm et 500 µm. Elle est dans l'exemple décrit égaie à 200 µm.

Les particules 9 sont dispersées dans le polymère constituant la matrice 2, comme illustré très schématiquement sur la figure 4.

La taille moyenne de ces particules 9 peut être comprise entre 5 et 70 µm par exemple et ces dernières peuvent faire saillie sur la face 3 extérieure de la matrice 2, destinée à être appliquée sur la peau.

Les particules 9 présentes à la surface de la matrice 2 sont alors enrobées d'une très fine couche du polymère constituant celle-ci.

Dans l'exemple décrit, le matériau hydrophile absorbeur d'eau utilisé est un polyacrylate hydroabsorbant, commercialisé par la Société ATO sous la dénomination AQUAKEEP, et sa proportion dans la matrice 2 est de 10% environ en poids.

La matrice 2 renferme dans l'exemple décrit les composés cosmétiquement actifs suivants, chacun étant d'une manière générale dans une proportion en poids inférieure à 6% : acide ascorbique (4%), menthol (0,05%), essence de lavande (1%), poudre de polyamide (3%), acide citrique (1%), aliantoine (1%).

Le patch présente un pouvoir adhésif sur peau sèche d'environ 500 g/cm² (force à exercer perpendiculairement au plan de la surface adhésive pour le décoller).

L'épaisseur du support 6 est de 2 mm dans l'exemple décrit, celle-ci étant d'une manière générale inférieure à 3 mm de préférence, de manière à ne pas rigidifier outre mesure le patch et a ne pas faire trop saillie sur la peau.

Pour utiliser le patch de la figure 1, on mouille le support 6 de manière à l'imbiber en totalité avec de l'eau ou avec une solution aqueuse telle qu'une lotion, comme illustré sur la figure 2, puis on retire la feuille de protection 4 et l'on applique le patch par la face extérieure 3 de la matrice 2 sur la zone à traiter, comme illustré sur la figure 3.

Dans l'exemple décrit, la peau est préalablement mouillée mais l'application pourrait se faire en variante sur peau sèche selon la nature du traitement à effectuer.

La matrice 2 est rendue perméable au liquide imprégnant le support 6 grâce à la présence des particules 9 du composé hydrophile absorbeur d'eau, l'eau traversant la matrice 2 en étant absorbée par ces particules 9, comme illustré très schématiquement sur la figure 4.

Les particules 9 qui se trouvent dans le voisinage de l'interface avec la peau, en se gorgeant d'eau, peuvent constituer entre la matrice 2 et la peau une couche intermédiaire gélifiée dans laquelle se solubilisent les composés cosmétiquement actifs, qui sont ainsi libérés au contact de l'épiderme.

Le mode de libération des composés cosmétiquement actifs par solubilisation dans le liquide déposé sur la peau et imprégnant le support 6 permet d'introduire dans la matrice 2 des composés cosmétiquement actifs qu'il serait impossible à conserver pendant une longue période dans une même solution.

Le support 6 retarde le séchage du patch à l'interface avec la peau, ce qui est favorable à une action prolongée des composés cosmétiquement actifs sur la peau et à une bonne diffusion de ceux-ci dans l'épiderme, le cas échéant.

Le support 6 imprégné d'eau procure également une impression de fraicheur à l'utilisateur.

Enfin, des impuretés ou toxines présentes à la surface de la peau peuvent être solubilisées à la surface de la matrice et diffuser dans cette dernière en direction du support 6 imprégné, sous l'effet de gradients de concentration.

Ainsi, la présence du support 6 imbibé d'eau peut favoriser le nettoyage de la peau.

Dans l'exemple décrit, on constate lors du retrait du patch au bout de 30 min environ, un éclaircissement de la zone traitée lié à la présence d'acide ascorbique et d'acide citrique. La peau a également gagné en souplesse et en douceur.

On peut encore, comme illustré sur la figure 5, réaliser des ajours dans la matrice 2 sans sortir du cadre de la présente invention.

Ces ajours peuvent être constitués par des microperforations 11 ou être obtenus en déposant la matrice 2 sur le support 6, sous la forme d'une grille à mailles plus ou moins fines.

Bien entendu, l'invention n'est pas limitée aux exemples de mise en oeuvre qui viennent d'être décrits.

On peut notamment utiliser un liquide comportant une ou plusieurs substances actives destinées à exercer un effet déterminé sur la peau, par exemple un anesthésique destiné à exercer une action d'insensibilisation locale.

Le support peut être un non-tissé à base de fibres de cellulose comme dans l'exemple décrit, mais peut aussi en variante être constitué par ou comporter un feutre, une mousse ou un matériau capable de par sa structure chimique de contenir le liquide utilisé, par exemple un matériau hydrogonflable.

## Revendications

1. Patch comportant une matrice présentant des propriétés adhésives à l'état sec et contenant au moins une substance active, cette matrice étant accolée par une face à un support et étant à appliquer par l'autre face sur la peau, ce patch étant **caractérisé par le fait que** ledit support (6) est choisi de manière à pouvoir contenir un liquide prédéterminé apte à solubiliser au moins partiellement la ou les substances actives contenues dans la matrice et **par le fait que** ladite matrice (2) est perméable à ce liquide, le liquide provoquant, au cours de sa diffusion à travers la matrice, le passage en solution de la ou des substances actives, lesquelles sont ainsi libérées de la matrice, le support étant imprégné par le liquide au moment de l'utilisation.

2. Patch selon la revendication 1, **caractérisé par le fait que** la matrice comporte un ou plusieurs polymères.

3. Patch selon la revendication 2, **caractérisé par le fait que** ledit liquide est de l'eau ou une solution aqueuse ou alcoolique, et **par le fait que** ladite matrice (2) comporte au moins un polymère hydrophobe.

4. Patch selon l'une quelconque des revendications 1 à 3, **caractérisé par le fait que** ledit support (6) est constitué d'une matière poreuse.

5. Patch selon la revendication 4, **caractérisé par le fait que** ledit support (6) est constitué par un non-tissé.

6. Patch selon l'une quelconque des revendications précédentes, **caractérisé par le fait que** la ou les substances actives contenues dans la matrice sont présentes sous forme particulaire.

7. Patch selon l'une quelconque des revendications précédentes, **caractérisé par le fait que** la matrice contient au moins une substance active qui est un composé cosmétiquement actif.

8. Patch selon la revendication précédente, **caractérisé par le fait que** ledit composé cosmétiquement actif est choisi parmi les agents émollients, hydratants, cicatrisants, régénérants, anti-rides, tenseurs, insensibilisants, protecteurs solaires, apaisants, auto-bronzants, éclaircissants, anti-tâches, réducteurs de graisse, ou des mélanges de ceux-ci.

9. Patch selon l'une quelconque des revendications précédentes, **caractérisé par le fait que** la matrice renferme une dispersion d'une charge de particules (9) d'au moins un composé absorbeur du liquide contenu dans le support (6).

10. Patch selon la revendication précédente, **caractérisé par le fait que** la teneur de la matrice en composé(s) absorbeur(s) est choisie de telle sorte que la quantité de liquide capable d'être absorbée par ce ou ces composé(s) soit supérieure ou égale à la quantité de liquide nécessaire à la solubilisation de la ou des substances actives contenues dans la matrice.

11. Patch selon l'une des revendications 9 et 10, **caractérisé par le fait que** le ou les composés absorbeurs sont choisis dans la liste suivante : polyacrylates réticulés superabsorbants à fort taux de gonflement dans l'eau, alcool polyvinylique, polymères carboxyvinyliques, dérivés semi-synthétiques de la cellulose, amidons, biogommes, biosaccharides, scléroglucanes, caséine, phytocolloïdes tels que les alginates, gélatine, fibres de coton, gélanes, xanthanes, laponite, silices ou des mélanges de ceux-ci.

12. Patch selon l'une quelconque des trois revendications précédentes, **caractérisé par le fait que** ladite charge est dans une proportion comprise entre 0,2% et 50% en poids, de préférence 1 à 20%, de préférence encore 1 à 10%, par rapport au poids total de la matrice.

13. Patch selon l'une quelconque des revendications précédentes, **caractérisé par le fait que** ladite matrice (2) comporte des ajours.

14. Patch selon la revendication précédente, **caractérisé par le fait que** ladite matrice comporte des microperforations (11).

15. Patch selon l'une quelconque des revendications précédentes, **caractérisé par le fait que** ladite matrice (2) est déposée sur ledit support (6) selon un motif en grille.

16. Patch selon l'une quelconque des revendications précédentes, **caractérisé par le fait que** ladite matrice renferme au moins une substance active capable de réagir chimiquement avec ledit liquide.

17. Ensemble constitué d'un patch tel que défini dans l'une quelconque des revendications 1 à 16, et d'un récipient contenant un liquide destiné à imprégner ledit support (6).

18. Ensemble selon la revendication précédente, **caractérisé par le fait que** le liquide comporte au moins une substance active destinée à exercer un effet prédéterminé sur la peau, tel qu'un anesthésique.

19. Procédé comportant les étapes consistant à :
- fournir un patch tel que défini dans l'une quelconque des revendications 1 à 16.
- imprégner avant ou après l'application du patch sur la peau ledit support (6) avec un liquide déterminé.

20. Procédé selon la revendication précédente, **caractérisé par le fait que** préalablement à l'application du patch sur la peau, on mouille cette dernière.

21. Procédé selon la revendication précédente, ledit liquide étant de l'eau, **caractérisé par le fait que** la quantité d'eau par unité de surface du support (6) qui imbibe ce dernier est supérieure à la quantité d'eau déposée sur la peau, par unité de surface.

22. Procédé selon l'une quelconque des revendications 19 à 21, **caractérisé par le fait que** l'on laisse le patch sur la peau pendant une durée comprise entre 5 min et 60 min.

## Patentansprüche

1. Pflaster, das eine Matrix enthält, die adhesive Eigenschaften im trockenen Zustand aufweist und die mindestens eine aktive Substanz beinhaltet, wobei diese Matrix mit einer Seitenfläche auf einen Träger geklebt und mit der anderen Seitenfläche an der Haut angewandt wird, wobei das Pflaster **dadurch gekennzeichnet, dass** der besagte Träger (6) so gewählt ist, dass er eine vorbestimmte Flüssigkeit enthalten kann, die in der Lage ist, die aktive(n) Substanz(en), die in der Matrix enthalten ist bzw. sind, wenigstens teilweise zu lösen, und dadurch, dass die besagte Matrix (2) für diese Flüssigkeit durchlässig ist, wobei die Flüssigkeit während ihrer Diffusion durch die Matrix das Übergehen der aktiven Substanz(en) in die Lösung verursacht, die somit von der Matrix abgegeben wird bzw. werden, worin der Träger im Moment der Anwendung mit der Flüssigkeit imprägniert ist.

2. Pflaster nach Anspruch 1, **dadurch gekennzeichnet, dass** die Matrix ein oder mehrere Polymere enthält.

3. Pflaster nach Anspruch 2, **dadurch gekennzeichnet, dass** die besagte Flüssigkeit Wasser oder eine wässrige oder alkoholische Lösung ist, und dass die besagte Matrix (2) mindestens ein hydrophobes Polymer enthält.

4. Plaster nach einem der Ansprüche 1 bis 3, **dadurch gekennzeichnet, dass** der besagte Träger (6) aus porösem Material beschaffen ist.

5. Pflaster nach Anspruch 4, **dadurch gekennzeichnet, dass** der besagte Träger (6) aus Vlies (nicht gewebten Gewebe) geschaffen ist.

6. Pflaster nach einem der vorhergehenden Ansprüche, **dadurch gekennzeichnet, dass** die aktive(n) Substanz(en), die in der Matrix beinhaltet ist bzw. sind, als Partikel vorliegt bzw. vorliegen.

7. Pflaster nach einem der vorhergehenden Ansprüche, **dadurch gekennzeichnet, dass** die Matrix mindestens eine aktive Substanz enthält, die eine kosmetisch aktive Verbindung ist.

8. Pflaster nach dem vorhergehenden Anspruch, **dadurch gekennzeichnet, dass** die besagte kosmetisch aktive Verbindung aus den aufweichenden, feuchtigkeitsspendenden, heilenden, regenerierenden, Anti-Falten-, spannenden, betäubenden, Sonnenschutz-, Beruhigungs-, Selbstbräunungs-, Aufhellungs-, Fleckenentfemungs-, und fettreduzierenden Mitteln oder aus Gemischen dieser ausgewählt wird.

9. Pflaster nach einem der vorhergehenden Ansprüche, **dadurch gekennzeichnet, dass** die Matrix eine Dispersion einer Charge von Partikeln (9) mindestens einer Zusammensetzung enthält, die die in dem Träger (6) enthaltene Flüssigkeit absorbiert.

10. Pflaster nach dem vorhergehenden Anspruch, **dadurch gekennzeichnet, dass** der Gehalt der Matrix an absorbierender / (-en) Zusammensetzung(en) so gewählt ist, dass die Menge an Flüssigkeit, die durch diese Zusammensetzung(en) absorbiert werden kann, größer oder gleich der Menge ist, die benötigt wird, um die aktive(n) Substanz(en), die in der Matrix enthalten ist bzw. sind, in Lösung zu bringen.

11. Pflaster nach einem der Ansprüche 9 oder 10, **dadurch gekennzeichnet, dass** die absorbierende(n) Zusammensetzung(en) aus der folgenden Liste ausgewählt wird bzw. werden: superabsorbierende, vernetzte, in Wasser stark anschwellende Polyacrylate, Polyvinylalkohole, Carboxyvinylpolymere, semisynthetische CelluloseDerivate, Stärken, Biogummen, Biosacchariden, Skleroglukanen, Casein, Phytocolloide wie z. B. Alginate, Gelatine, Baumwollfasern, Gélane, Xanthane, Laponite, Kieselerde oder Gemische derselben.

12. Pflaster nach einem der drei letztgenannten Ansprüche, **dadurch gekennzeichnet, dass** die besagte Charge in einer Menge zwischen 0,2 und 50 Gew.-%, bevorzugterweise 1 bis 20 %, stärker bevorzugt zwischen 1 bis 10 %, bezogen auf das Gesamtgewicht der Matrix, vorliegt.

13. Pflaster nach einem der vorhergehenden Ansprüche, **dadurch gekennzeichnet, dass** die besagte Matrix (2) Öffnungen aufweist.

14. Pflaster nach dem vorhergehenden Anspruch, **dadurch gekennzeichnet, dass** die besagte Matrix Mikroperforationen aufweist (11).

15. Pflaster nach einem der vorhergehenden Ansprüche, **dadurch gekennzeichnet, dass** die besagte Matrix (2) auf dem besagten Träger (6) gitterförmig abgelegt ist.

16. Pflaster nach einem der vorhergehenden Ansprüche, **dadurch gekennzeichnet, dass** die besagte Matrix mindestens eine aktive Substanz enthält, die mit der besagten Flüssigkeit chemisch reagieren kann.

17. Satz bzw. Ensemble, der bzw. das aus einem Pflaster, wie es in einem der vorhergehenden Ansprüche 1 bis 16 definiert wurde, und aus einem Behälter, der eine Flüssigkeit enthält, die dazu bestimmt ist, den besagten Träger (6) zu imprägnieren, besteht.

18. Satz bzw. Ensemble nach dem vorhergehenden Anspruch, **dadurch gekennzeichnet, dass** die Flüssigkeit mindestens eine aktive Substanz enthält, die dazu bestimmt ist, einen vorherbestimmten Effekt auf die Haut auszuüben, wie z. B. ein Anästhetikum.

19. Verfahren, umfassend die Schritte, bestehend aus:
- Bereitstellen eines Pflasters, wie es in einem der Ansprüche 1 bis 16 definiert wurde,
- Imprägnieren des besagten Trägers (6) vor oder nach der Anwendung des Pflasters auf der Haut mit einer bestimmten Flüssigkeit.

20. Verfahren nach dem vorhergehenden Anspruch, **dadurch gekennzeichnet, dass** vor der Anwendung des Pflasters auf der Haut letzere angefeuchtet wird.

21. Verfahren nach dem vorhergehenden Anspruch, wobei die besagte Flüssigkeit Wasser ist, **dadurch gekennzeichnet, dass** die Wassermenge pro Oberflächeneinheit des Trägers (6), die von letzterem aufgenommen wird, größer ist als die Menge an Wasser, die pro Oberflächeneinheit auf die Haut abgegeben wird.

22. Verfahren nach einem der vorhergehenden Ansprüche 19 bis 21, **dadurch gekennzeichnet, dass** man das Pflaster während einer Zeitspanne zwischen 5 Minuten und 60 Minuten auf der Haut lässt.

## Claims

1. A patch comprising a matrix having adhesive properties in the dry state and containing at least one active substance, said matrix being attached via one face to a backing and being for application via its other face to the skin, the patch being **characterized by** the fact that said backing (6) is selected in such a manner as to be capable of containing a predetermined liquid suitable for dissolving at least in part the active substance(s) contained in the matrix, and by.the fact that said matrix (2) is permeable to the liquid, the liquid causing, as it diffuses through the matrix, the active substance(s) to pass into solution, the active substance(s) being thereby released from the matrix, the backing being impregnated with the liquid when used.

2. A patch according to claim 1, **characterized by** the fact that the matrix comprises one or more polymers.

3. A patch according to claim 2, **characterized by** the fact that said liquid is water or an aqueous or alcohol solution, and by the fact that said matrix (2) comprises at least one hydrophobic polymer.

4. A patch according to any one of claims 1 to 3, **characterized by** the fact that said backing (6) is constituted by a porous material.

5. A patch according to claim 4, **characterized by** the fact that said backing (6) is constituted by a non-woven cloth.

6. A patch according to any preceding claim, **characterized by** the fact that the active substance(s) contained in the matrix is/are present in particulate form.

7. A patch according to any preceding claim, **characterized by** the fact that the matrix contains at least one active substance which is a cosmetically active compound.

8. A patch according to the preceding claim, **characterized by** the fact that the cosmetically active compound is selected from: emollients, moisturizers, healing agents, regenerating agents, anti-wrinkle agents, tightening agents, anesthetics, sun screens, soothing agents, self-tanning agents, brightening agents, concealers, grease reducers, and mixtures thereof.

9. A patch according to any preceding claim, **characterized by** the fact that the matrix contains in dispersion a filler of particles (9) of at least one compound that absorbs the liquid contained in the backing (6) .

10. A patch according to the preceding claim, **characterized by** the fact that the concentration in the matrix of the absorbing compound(s) is selected in such a manner that the quantity of liquid that can be absorbed thereby is greater than or equal to the quantity of liquid required for dissolving the active substance(s) contained in the matrix.

11. A patch according to claim 9 or 10, **characterized by** the fact that the absorbing compound(s) is/are selected from the following list: superabsorbent cross-linked polyacrylates having a large swelling factor in water, polyvinyl alcohol, carboxyvinyl polymers, semi-synthetic derivatives of cellulose, starches, biogums, biosaccharides, scleroglucanes, casein, photocolloids such as alginates, gelatin, cotton fibers, gellanes, xanthanes, laponite, silicas, or mixtures thereof.

12. A patch according to any of the three preceding claims, **characterized by** the fact that said filler is at a concentration lying in the range 0.2% to 50% by weight, preferably in the range 1% to 20%, more preferably in the range 1% to 10% relative to the total weight of the matrix.

13. A patch according to any preceding claim, **characterized by** the fact that said matrix (2) has gaps.

14. A patch according to the preceding claim, **characterized by** the fact that said matrix has microperforations (11).

15. A patch according to any preceding claim, **characterized by** the fact that said matrix (2) is deposited on said backing (6) in a grid pattern.

16. A patch according to any preceding claim, **characterized by** the fact that said matrix contains at least one active substance capable of reacting chemically with said liquid.

17. A kit constituted by a patch as defined in any one of claims 1 to 16, and a receptacle containing a liquid for impregnating said backing (6).

18. A kit according to the preceding claim, **characterized by** the fact that the liquid includes at least one active substance for exercising a predetermined effect on the skin, such as an anesthetic.

19. A method comprising the steps consisting in:
· supplying a patch as defined in any of claims 1 to 16; and
· impregnating said backing (6) with a determined liquid before or after applying the patch to the skin.

20. A method according to the preceding claim, **characterized by** the fact that prior to application of the patch on the skin, the skin is wetted.

21. A method according to the preceding claim, said liquid being water, the method being **characterized by** the fact that the quantity of water per unit area of the backing (6) that soaks up the water is greater than the quantity of water deposited on the skin, per unit area.

22. A method according to any one of claims 19 to 21, **characterized by** the fact that the patch is left on the skin for a length of time lying in the range 5 min to 60 min.
